# EUROPÄISCHE PATENTSCHRIFT

(11) **EP 1 000 632 B1**
(45) Veröffentlichungstag und Bekanntmachung des Hinweises auf die Patenterteilung: **08.06.2005**
(21) Anmeldenummer: 99120405.8
(22) Anmeldetag: 13.10.1999
(51) Int. Cl.: A61M 39/20, A61M 1/16, B01D 61/30

(54) **Verschlusselement**
Closure element
Elément de fermeture

(30) Priorität: 13.11.1998 DE 19852557
(43) Veröffentlichungstag der Anmeldung: 17.05.2000
(73) Patentinhaber: Fresenius Medical Care Deutschland GmbH, 61352 Bad Homburg v.d.H. (DE)
(72) Erfinder: Hahmann, Uwe, 61352 Bad Homburg (DE); Heilmann, Klaus, 66606 St. Wendel (DE); Fritzsche, Steffen, Dr., 66636 Tholey (DE); Sauerwald, Christian, 66606 St. Wendel (DE); Breith, Gerhard, 66953 Pirmasens (DE); Raiko, Igor, 66606 St. Wendel (DE)
(74) Vertreter: Laufhütte, Dieter, Dr.-Ing.

(56) Entgegenhaltungen:
- EP-A- 0 352 540
- US-A- 4 387 879
- US-A- 5 817 043

## Beschreibung

Die vorliegende Erfindung betrifft einem medizinischen Artikel bestehend aus einem Filtermodul für die Dialyse, Hämo- oder Ultrafiltration.

Bei der Herstellung derartiger Filtermodule besteht die Notwendigkeit, diese nach dem eigentlichen Herstellprozeß zu sterilisieren und ferner vor äußeren Einflüssen, wie z.B. dem Eindringen von Keimen oder Schmutzpartikeln, zu schützen. Ein Verfahren zur Sterilisation von Filtermodulen ist die sogenannte Inline-Sterilisation, bei der die innenliegenden Bereiche des Filtermoduls gereinigt werden, während eine Sterilisation an der Außenseite nicht erfolgt. Da jeder offene Kontakt der Anschlüsse des Filtermoduls bei oder nach der Durchführung des Sterilisationsschrittes zwingend zu vermeiden ist, muß das Filtermodul derart abgedichtet werden, daß weder während der Sterilisation noch nach der Abnahme von der Sterilisationsvorrichtung eine Kontamination oder Verschmutzung des Innenraums des Filtermoduls erfolgen kann.

Aus der EP 0 352 540 ist ein gattungsgemäßer Artikel mit einem Verschlußelement bekannt, das aus zwei Teilen besteht, von denen das erste Verschlußelementteil an dem Anschluß des Filtermoduls bzw. Dialysators angebracht wird, während das zweite Verschlußelementteil in einem Aufnahmeraum des ersten Verschlußelementteils aufgenommen ist. Es ist in diesem Aufnahmeraum zwischen einer Offenstellung und einer Schließstellung bewegbar angeordnet. Somit läßt sich in der Offenstellung ein Durchtritt des Sterilisationsmediums bzw. die Sterilisation sämtlicher Dichtund Führungsflächen erreichen und in der Schließstellung die zuverlässige Abdichtung des Dialysators erzielen. Ein derartiges Verschlußelement gewährleistet zwar die zuverlässige Abdichtung des Filtermoduls, weist jedoch den Nachteil eines verhältnismäßig komplexen Aufbaus auf.

Es ist daher die Aufgabe der vorliegenden Erfindung, einen gattungsgemäßen Artikel dahingehend weiterzubilden, daß dieser einfach aufgebaut ist und zuverlässig einen sterilen Verschluß gewährleistet.

Diese Aufgabe wird ausgehend von einem Artikel mit den Merkmalen des Oberbegriffs des Anspruchs 1 dadurch gelöst, daß das Verschlußelement eine Wandung umfaßt, die einen selbsttätig schließenden schlitzförmigen Einschnitt aufweist, der im geschlossenen Zustand keimdicht abschließt, und daß Befestigungsmittel vorgesehen sind, die an die Wandung angrenzen und mittels derer das Verschlußelement mit einem Anschluß abdichtend verbindbar ist. Ein derartiges Verschlußelement ist einfach aufgebaut und bewirkt aufgrund des selbsttätig schließenden schlitzförmigen Einschnittes eine zuverlässige und sterile Abdichtung eines medizinischen Artikels bestehend aus einem Filtermodul, während und auch nach der Sterilisation. Beim Einführen eines entsprechenden Konnektionselementes, beispielsweise eines Stutzens einer Sterilisationsvorrichtung oder einer Dialysemaschine, wird der schlitzförmige Einschnitt des erfindungsgemäßen Verschlußelementes entsprechend der Form des Konnektionselementes geringfügig oder auch vollständig geöffnet.

Wird das Konnektionselement nach der erfolgten Sterilisation oder Behandlung aus dem erfindungsgemäßen Verschlußelement entfernt, schließt der schlitzförmige Einschnitt selbsttätig und verhindert somit die Kontamination des sterilisierten Bereiches sowie zusätzlich das Auslaufen des mit dem erfindungsgemäßen Verschlußelement verschlossenen Artikels.

Besonders vorteilhaft ist es, wenn das Verschlußelement eine im wesentlichen zylindrische Gestalt aufweist, wobei die Befestigungsmittel durch die Zylinderfläche gebildet werden und der schlitzförmige Einschnitt in einer der Stirnflächen des Zylinders angeordnet ist. Mittels der Zylinderfläche wird das Verschlußelement keimdicht mit einem Anschluß des medizinisches Artikels verbunden. Eine derartige, kappenartige Ausgestaltung des Verschlußelementes weist den Vorteil auf, daß die zu- bzw. abgeführte Flüssigkeit beim Durchtritt durch das Verschlußelement eine einfache, geradlinige Bahn durchläuft, wodurch bei entsprechender Anordnung des schlitzförmigen Einschnittes die Bildung von Totzonen wirkungsvoll vermieden werden kann. Daraus ergibt sich der Vorteil, daß eine Vermischung unterschiedlicher Flüssigkeiten, wie z.B. unterschiedliche Wirkstoffe oder Medikamente enthaltende Gemische, durch unterschiedliche Verweilzeiten in dem Verschlußelement vermieden werden kann.

In weiterer Ausgestaltung der vorliegenden Erfindung ist vorgesehen, daß das Verschlußelement symmetrisch ausgeführt ist. Ein derartiger Aufbau vereinfacht nicht nur die Herstellung des Verschlußelementes, sondern ermöglicht darüber hinaus einen einfachen Umgang bzw. ein einfaches Auf- und Einsetzen des Verschlußelementes auf den entsprechenden Anschluß beispielsweise eines Filtermoduls.

Gemäß einer bevorzugten Ausgestaltung der vorliegenden Erfindung ist vorgesehen, daß der schlitzförmige Einschnitt kreuz- oder sternförmig ausgeführt ist. Dadurch wird gegenüber einer einfachen gradlinigen Ausgestaltung des Einschnittes eine sehr gute Flexibilität und Anpassung an Konnektionsstutzen variabler Form oder variierender Durchmesser ermöglicht.

Es kann eine der den Einschnitt aufweisenden Wandung gegenüberliegende weitere Wandung vorgesehen sein, die eine Öffnung zum Durchtritt eines Fluids aufweist. Dabei können der mittlere Bereich des Einschnittes und der Öffnung miteinander fluchten.

In weiterer Ausgestaltung der Erfindung ist vorgesehen, daß der sich um die Öffnung der weiteren Wandung erstreckende Bereich in einer im wesentlichen senkrecht zur Fügerichtung eines mit dem Anschluß verbindbaren Konnektionselementes verlaufenden Ebene angeordnet ist. Hieraus ergibt sich der Vorteil, daß das erfindungsgemäße Verschlußelement zusätzlich zur dichten Verbindung des Konnektionselementes, beispielsweise der Dialysemaschine, und des Filtermoduls dient. Eine Abdichtung wird dadurch erreicht, daß das Konnektionselement mit seiner Stirnseite dichtend auf der inneren Seite der weiteren Wandung aufsetzt.

Besonders vorteilhaft ist es, wenn das erfindungsgemäße Verschlußelement einteilig ausgeführt ist. Hierdurch ergeben sich entsprechende Vorteile bei dem Herstellvorgang.

Besonders vorteilhaft ist es, wenn die den Einschnitt aufweisende Wandung oder das gesamte Verschlußelement aus Kunststoff bestehen. Daraus ergibt sich zum einen der Vorteil, daß durch die Wahl eines geeigneten Kunststoffes die Flexibilität und Elastizität des Verschlußelementes in weiten Bereichen variierbar ist. Darüber hinaus läßt sich durch die Wahl des Kunststoffes die Kompatibilität mit der eingesetzten Flüssigkeit bzw. mit einem unterschiedliche Wirkstoffe enthaltenden Gemisch erreichen. Die Verwendung von Kunststoff für die den Einschnitt aufweisende Wandung des Verschlußelementes oder für das gesamte Verschlußelement weist ferner den Vorteil auf, daß aufgrund des einfachen und kostengünstigen Herstellverfahrens eine Vielfalt von unterschiedlichen Ausführungsformen und Ausgestaltungen des Verschlußelementes herstellbar ist. Besonders vorteilhaft ist es, wenn der Kunststoff Silikon ist.

Besonders vorteilhaft ist es, wenn die den schlitzförmigen Einschnitt aufweisende Wandung des Verschlußelementes ein in radialer Richtung wirkendes Federelement aufweist. Hierdurch ergibt sich der Vorteil, radiale Spannungskräfte über die Querrichtung im Federelementverlauf abzusenken. Dadurch wird erreicht, daß der schlitzförmige Einschnitt der Wandung ein verbessertes Dichtverhalten aufweist.

In weiterer Ausgestaltung der vorliegenden Erfindung ist vorgesehen, daß das Federelement aus abwechselnd oder einzelnen auf der Ober- und/oder Unterseite der Wandung angeordneten Vertiefungen gebildet wird. Die Vertiefungen können teilkreisförmig ausgeführt sein.

Besonders vorteilhaft ist es, wenn in den sich in Längsrichtung des schlitzförmigen Einschnittes erstreckenden Bereichen der Wandung kein Federelement vorgesehen ist. Dadurch wird die Radialkrafteinwirkung in Schlitzrichtung erhöht, was eine besonders dichte Ausgestaltung des schlitzförmigen Einschnittes ergibt.

In weiterer Ausgestaltung der vorliegenden Erfindung ist vorgesehen, daß der schlitzförmige Einschnitt des Verschlußelementes bis zu einer Druckdifferenz von +/- 0,25 bar keimdicht schließt.

Die vorliegende Erfindung betrifft ferner die Verwendung eines erfindungsgemäßen Verschlußelementes für den sterilen Verschluß von Anschlüssen medizinischer Artikel, wobei das Verschlußelement für die Inline-Sterilisation des Filtermoduls verwendet wird.

In weiterer Ausgestaltung der vorliegenden Erfindung ist vorgesehen, daß das Befestigungsmittel des Verschlußelementes derart ausgeführt ist, daß das Verschlußelement auf vorstehende oder in buchsenförmige Anschlüsse auf- bzw. einsetzbar ist.

Besonders vorteilhaft ist es, wenn das Verschlußelement derart ausgeführt ist, daß die Innenfläche des Verschlußelementes mit der Außenfläche eines vorstehenden Anschlusses oder die Außenfläche des Verschlußelementes mit der Innenfläche eines buchsenförmigen Anschlusses keimdicht verbunden sind.

In weiterer Ausgestaltung der vorliegenden Erfindung ist vorgesehen, daß mindestens zwei Anschlüsse des medizinischen Artikels mit einem erfindungsgemäßen Verschlußelement versehen sind.

Weitere Einzelheiten und Vorteile der vorliegenden Erfindung werden anhand von in der Zeichnung dargestellten Ausführungsbeispielen näher erläutert.

Es zeigen:
- Fig. 1:: einen Längsschnitt durch ein erfindungsgemäßes Verschlußelement gemäß einer ersten Ausführungsform sowie durch eine entsprechende Gehäuseaussparung zur Aufnahme des Verschlußelementes;
- Fig. 2:: eine Ansicht des Verschlußelementes nach Fig. 1 gemäß Perspektive A-A (links) und Perspektive B-B (rechts);
- Fig. 3:: einen Längsschnitt des eingesetzten Verschlußelementes nach Fig. 1 während eines Sterilisationsvorganges;
- Fig. 4:: einen Längsschnitt des eingesetzten Verschlußelementes nach Fig. 1 während der bestimmungsgemäßen Benutzung;
- Fig. 5:: eine Draufsicht auf ein erfindungsgemäßes Verschlußelement mit Federelementen gemäß einer zweiten Ausführungsform;
- Fig. 6:: einen Längsschnitt des Verschlußelementes nach Fig. 5 gemäß der Linie A-A und
- Fig. 7:: einen Längsschnitt des eingesetzten erfindungsgemäßen Verschlußelementes gemäß Fig. 5 unmittelbar vor der Verbindung mit einem Konnektionselement.

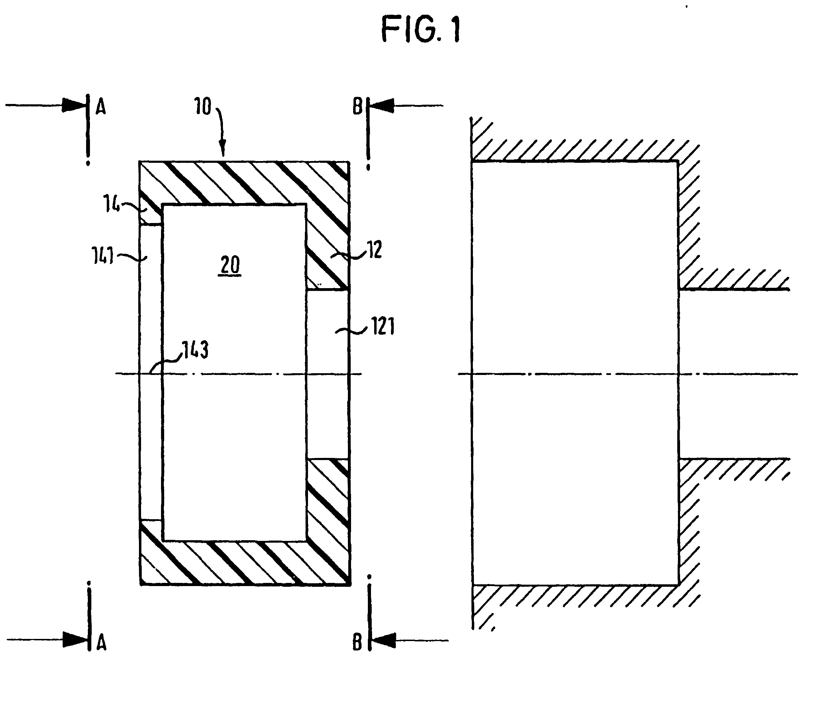

Fig. 1 zeigt das erfindungsgemäße Verschlußelement 10 mit den parallel verlaufenden Wandungen 12 und 14. Beide Wandungen 12, 14 begrenzen den zylindrischen Hohlraum 20, der ferner durch die sich an die Wandungen 12, 14 anschließenden und als Befestigungsmittel dienenden Zylinderflächen festgelegt wird. Die Wandungen 12 und 14 grenzen rechtwinklig an die Zylinderflächen des Verschlußelementes 10 an.

Die Wandung 14 weist den schlitzförmigen und selbsttätig schließenden Einschnitt 141 mit dem zentralen Abschnitt 143 auf. In der Wandung 12 befindet sich zentrisch die Öffnung 121, die im vorliegenden Ausführungsbeispiel kreisrund ausgeführt ist. Die Öffnung 121 sowie der zentrale Abschnitt 143 des Einschnittes 141 der Wandung 14 fluchten miteinander, so daß das vorliegende Verschlußelement für die Verwendung von geradlinigen Konnektionselementen einsetzbar ist.

Fig. 1, rechts zeigt eine Gehäuseöffnung, beispielsweise eines Filtermoduls, zur Aufnahme des erfindungsgemäßen Verschlußelementes 10. Diese ist vorteilhaft ebenfalls zylindrisch ausgeführt und nimmt vor Betriebsbeginn das Verschlußelement 10 formschlüssig auf. Um einen zuverlässigen Sitz des Verschlußelementes in der Gehäuseaussparung zu erreichen, liegt der Außendurchmesser des Verschlußelementes 10 geringfügig über dem Innendurchmesser der Gehäuseaussparung. Damit wird außerdem eine sichere und keimdichte Abdichtung zwischen Gehäuse und Verschlußelement 10 erzeugt.

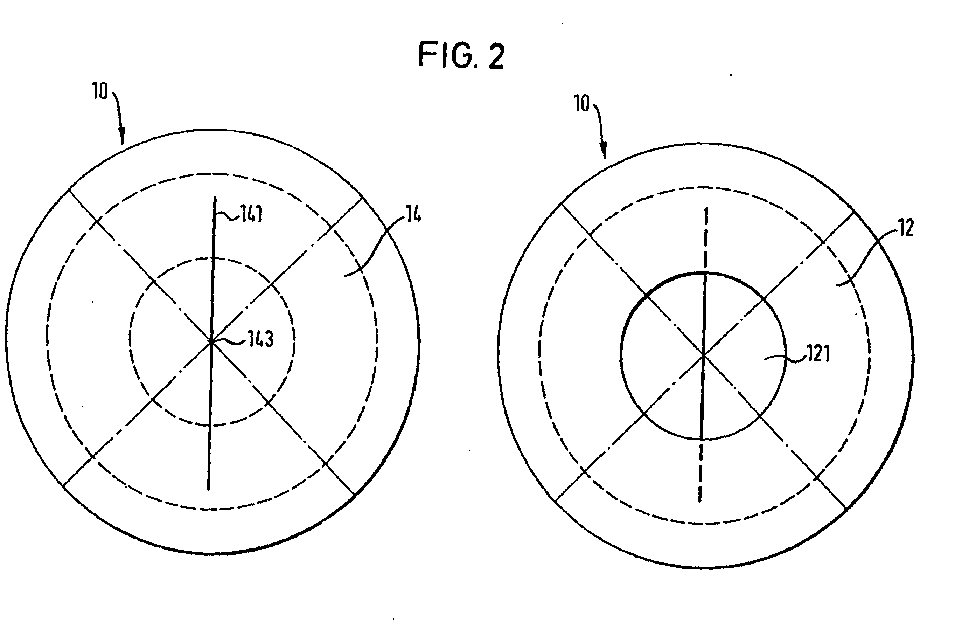

Fig. 2 zeigt eine Ansicht des erfindungsgemäßen Verschlußelementes 10 aus der Perspektive A-A (links) und B-B (rechts) gemäß Fig. 1. Die durchgezogenen Linien in Fig. 2 entsprechen den sichtbaren Konturen des Verschlußelementes 10, während die durchbrochenen Linien Schnittkanten darstellen, die in der gewählten Perspektive nicht sichtbar sind. Fig. 2, links zeigt das Verschlußelement 10 mit der Wandung 14, die den selbsttätig schließenden schlitzförmigen Einschnitt 141 mit einem zentralen Abschnitt 143 aufweist. Die Zylinderflächen des Hohlzylinders sind in Fig. 2 durch die äußerste durchgezogene sowie die nach innen versetzte durchbrochene Linie dargestellt. Fig. 2, rechts zeigt die der Wandung 14 gegenüberliegende Wandung 12. In dieser befindet sich die kreisförmige und zentrische Öffnung 121.

Fig. 2 verdeutlicht, daß die Öffnung 121 sowie der zentrale Abschnitt 143 des schlitzförmigen Einschnittes 141 miteinander fluchten. Der schlitzförmige Einschnitt 141 ist in Fig. 2, rechts als teilweise durchbrochene Linie dargestellt, da dieser in der gewählten Ansicht nur innerhalb der Öffnung 121 sichtbar ist.

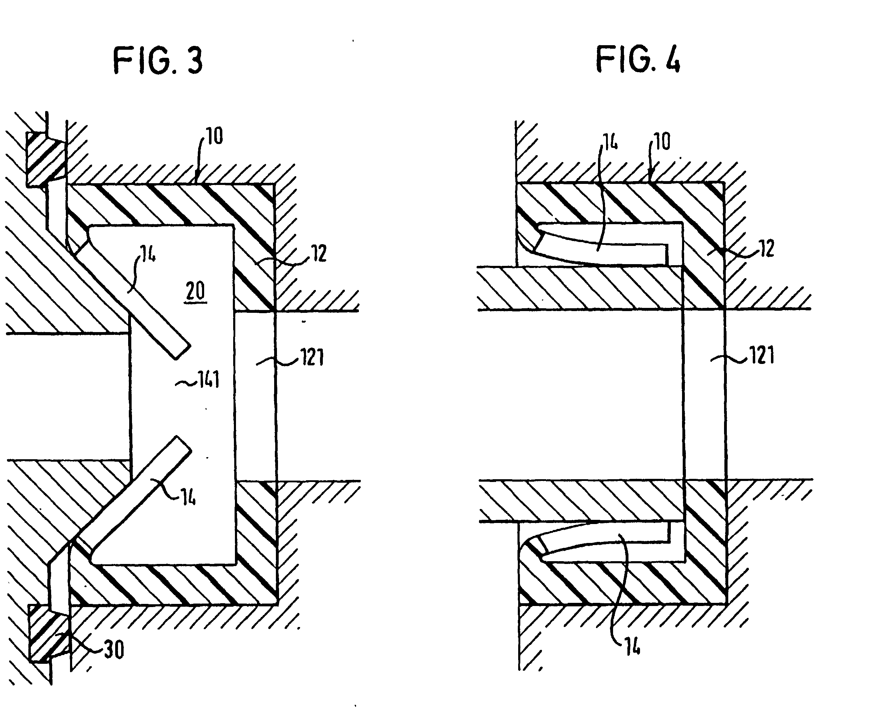

Fig. 3 zeigt das erfindungsgemäße Verschlußelement 10 während eines Sterilisationsprozesses. Der gemäß Fig. 3 von links eingeführte Stutzen des Sterilisationsgerätes wird derart eingeführt, daß er zunächst die Wandung 14 berührt und beim weiteren Einführen die elastisch ausgeführte Wandung 14 derart verformt, daß sich der im nicht benutzten Zustand geschlossene schlitzförmige Einschnitt 141 aufweitet und eine Öffnung für das ein- bzw. ausströmende Sterilisiermedium bildet, was als Flüssigkeit, Gas oder Dampf ausgebildet sein kann. Gemäß dem in Fig. 3 gezeigten Ausführungsbeispiel erfolgt eine Abdichtung mittels eines am Sterilisationsgerät befindlichen Dichtringes 30, der über das das Verschlußelement 10 aufnehmende Gehäuse abdichtet. Die Sterilisationsflüssigkeit tritt nun durch die Öffnung 141 in den Hohlraum 20 des Verschlußelementes 10 sowie in den Ringbereich ein, der sich bis zu dem am Gehäuse anliegenden Dichtring 30 erstreckt. Nach Passieren des zu einer Öffnung verformten schlitzförmigen Einschnittes 141 gelangt die Flüssigkeit durch die Öffnung 121 in das zu sterilisierende Bauteil, beispielsweise in einen Dialysefilter. Nach Entnahme des Sterilisationsgerätestutzens bei Beendigung des Prozesses wird die Wandung 14 des Verschlußelementes 10 in ihre ursprüngliche Gestalt zurückverformt, wodurch sich die Öffnung schließt und sich erneut ein schlitzförmiger Einschnitt 141 bildet. Dieser schließt nunmehr das sterilisierte Bauteil dicht ab, wodurch zum einen das Auslaufen und Abtropfen beispielsweise von Sterilisationsflüssigkeit und zum anderen das Eindringen unerwünschter Substanzen und Keime verhindert wird.

In Fig. 4 ist das erfindungsgemäße Verschlußelement 10 während der bestimmungsgemäßen Benutzung dargestellt. Hierbei wird ein Konnektionsstutzen derart durch den schlitzförmigen Einschnitt 141 der Wandung 14 eingeführt, bis die Stirnseite des Konnektionsstutzens an der zum Hohlraum 20 des Verschlußelementes 10 angrenzenden Seite der Wandung 12 abdichtend anliegt. In Abhängigkeit des Durchmessers des Konnektionsstutzens werden dabei die auf beiden Seiten des schlitzförmigen Einschnittes 141 befindlichen Bereiche der Wandung 14 in Richtung der Wandung 12 gekrümmt, wie dies aus Fig. 4 ersichtlich ist. Die zu- oder abzuführende Flüssigkeit gelangt nun durch die Öffnung des Konnektionsstutzens und durch die Öffnung 121 der Wandung 12 in bzw. aus dem konnektierten Bauteil, beispielsweise einem angeschlossenen Dialysefilter. Durch den dichten Abschluß der Stirnseite des Konnektionsstutzens an der Innenseite der Wandung 12 wird eine dichte und somit keimfreie Konnektion erreicht. Darüber hinaus verdeutlicht Fig. 4, daß bei entsprechend günstiger Abstimmung von Konnektionsstutzen und Verschlußelement eine geradlinige Durchführung für die Flüssigkeit erreicht wird, wodurch die Bildung von unerwünschten Totzonen weitgehend vermieden werden kann.

Bei Beendigung der Behandlung wird der Konnektionsstutzen zurückgezogen, wodurch sich die Bereiche der Wandung 14 in ihre ursprüngliche Gestalt zurückverformen und wie in Fig. 2 dargestellt erneut einen dicht abschließenden schlitzförmigen Einschnitt 141 bilden.

Die in Fig. 3 und Fig. 4 dargestellten Betriebsformen verdeutlichen, daß weder bei der Sterilisation noch bei dem bestimmungsgemäßen Gebrauch des erfindungsgemäßen Verschlußelementes 10 Keime in den Kreislauf bzw. in das konnektierte Bauteil gelangen können. Ferner besteht ein Vorteil darin, daß das erfindungsgemäße Verschlußelement 10 aus der entsprechenden Gehäuseaussparung leicht entnommen werden kann, wodurch ein einfaches sowie billiges Auswechseln möglich ist. Auf diese Weise wird eine stets hygienische sowie sterile Konnektion der zu verbindenden Bauteile erreicht, und eine Verunreinigung der zu fördernden Flüssigkeiten vermieden.

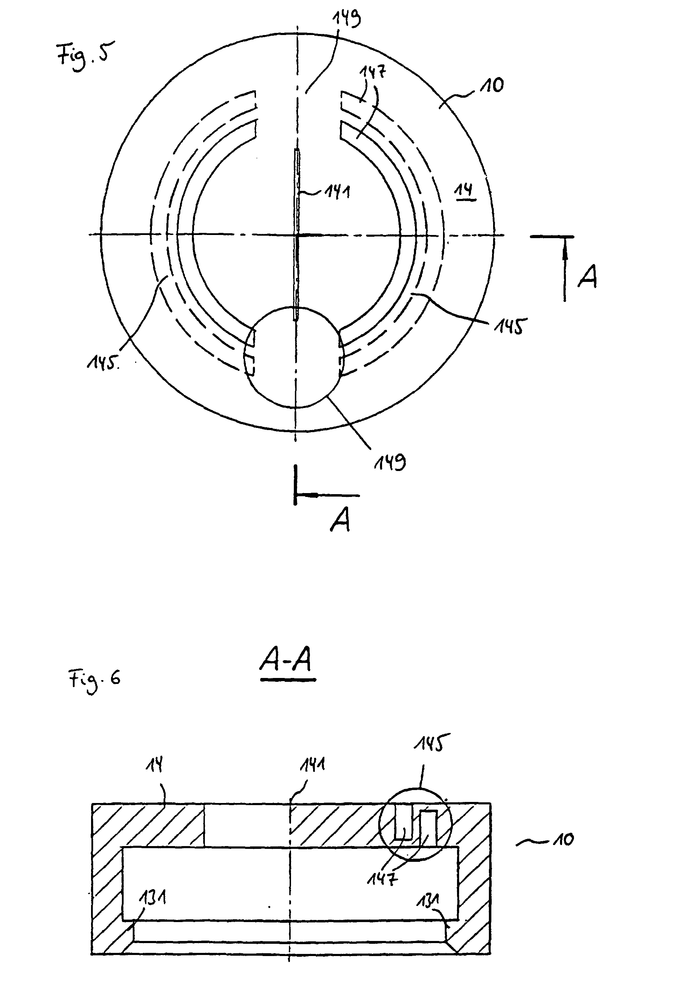

Die Fig. 5-7 zeigen eine zweite Ausführungsform des erfindungsgemäßen Verschlußelementes 10.

In Fig. 5 ist eine Draufsicht auf die zweite Ausführungsform des Verschlußelementes 10 dargestellt. Das Verschlußelement 10 weist eine zylinderförmige Gestalt auf, wobei in der in Fig. 5 ersichtlichen Stirnfläche der selbsttätig schließende schlitzförmige Einschnitt 141 der Wandung 14 erkennbar ist. In den sich rechts und links des schlitzförmigen Einschnittes 141 erstreckenden Bereichen sind die Federelemente 145 angeordnet. Diese dienen dazu, die radialen Spannungskräfte in Querrichtung im Federelementverlauf durch eine Querschnittsverringerung der Wandung 14 abzusenken.

Wie sich insbesondere aus der Längsschnittdarstellung gemäß Fig. 6 ergibt, besteht das Federelement 145 gemäß dem vorliegenden Ausführungsbeispiel aus abwechselnd aus der Ober- und Unterseite der Wandung 14 angeordneten Vertiefungen 147. Die sich daraus ergebenden Verringerungen der Querschnittsfläche der Wandung 14 führen zu der gewünschten Federwirkung.

Fig. 5 verdeutlicht, daß sich die Federelemente 145 nicht vollständig, sondern teilkreisförmig um den schlitzförmigen Einschnitt 141 der Wandung 14 erstrecken und in dem sich in Längsrichtung des Einschnittes 141 erstreckenden Bereich 149 nicht vorgesehen sind. Hieraus ergibt sich der Vorteil, daß die Radialkrafteinwirkung in Schließrichtung erhöht wird, wodurch sich ein besonders wirksamer und keimdichter Verschluß des Einschnittes 141 ergibt. Eine derartige konstruktive Ausgestaltung des Verschlußelementes mit Federelementen 145 führt somit dazu, daß sich ein verbessertes Dichtverhalten des Einschnittes 141 der Wandung 14 ergibt.

Die Federbereiche 145 können vorteilhaft derart ausgeführt werden, daß bei Ausführung des Verschlußelementes 10 aus Silikon entsprechend dem vorliegenden Ausführungsbeispiel im unbelasteten Zustand eine Druckdifferenz von +/- 0,25 bar tolerierbar ist, ohne daß der Einschnitt 141 seine Dichtwirkung verliert.

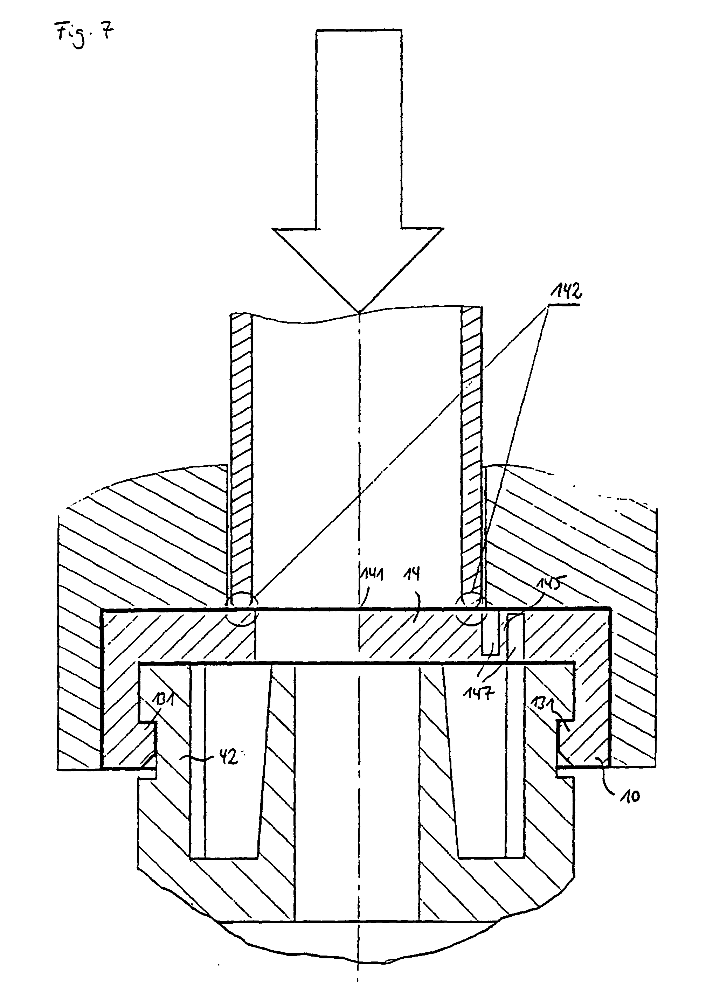

Aus der Längsschnittdarstellung gemäß Fig. 6 geht weiterhin hervor, daß der sich von der Wandung 14 senkrecht erstreckende und als Befestigungsmittel dienende Zylindermantel in seinem Endbereich den umlaufenden Vorsprung 131 aufweist. Dieser dient dazu, das Verschlußelement 10 in eine entsprechende Aussparung des in Fig. 7 dargestellten Anschlußstutzens 42 einzurasten bzw. sicher dort zu fixieren.

Fig. 7 zeigt in einem Längsschnitt das eingesetzte Verschlußelement 10 in der Ausführungsform gemäß Fig. 5 und 6. Das Verschlußelement 10 ist hier auf den vorstehenden Anschlußstutzen 42 aufgesetzt und mittels des umlaufenden Vorsprungs 131 arretiert.

Wird im Bereich 142 der Wandung 14 eine Druckkraft in Pfeilrichtung aufgebracht, die mechanisch und/oder pneumatisch und/oder hydraulisch erzeugt werden kann, wird der Einschnitt 141 geringfügig oder vollständig geöffnet.

Um eine sterile Abdichtung zu gewährleisten ist es erforderlich, daß nicht nur der schlitzförmige Einschnitt 141 der Wandung 14 dicht abschließt, sondern daß sich auch eine abdichtende Verbindung zwischen Verschlußelement 10 und dem Anschlußstutzen 42 ergibt. Dies wird dadurch erreicht, daß das Verschlußelement 10 derart ausgeführt ist, daß seine das Befestigungsmittel bildende Zylinderfläche sowohl auf der Außen- als auch auf der Innenseite auf Passung ausgeführt ist. Daraus ergibt sich, daß das aufgesetzte bzw. eingesetzte Verschlußelement 10 über die Innenfläche mit der Außenfläche eines vorstehenden Anschlußstutzens 42 bzw. über die Außenfläche mit der Innenfläche eines buchsenförmigen Anschlußstutzens einen keimdichten Verschluß gewährleistet.

## Patentansprüche

1. Medizinischer Artikel bestehend aus einem Filtermodul für die Dialyse, Hämo- oder Ultrafiltration mit einem oder mehreren Anschlüssen (42) zur Zu- und/oder Abführung eines Fluids, wobei mindestens ein Anschluß (42) mit einem Verschlußelement (10) versehen ist, **dadurch gekennzeichnet, daß** das Verschlußelement (10) eine Wandung (14) umfaßt, die einen selbsttätig schließenden schlitzförmigen Einschnitt (141) aufweist, der im geschlossenen Zustand keimdicht abschließt, und daß Befestigungsmittel vorgesehen sind, die an die Wandung (14) angrenzen und mittels derer das Verschlußelement (10) mit dem Anschluß (42) abdichtend verbindbar ist.

2. Medizinischer Artikel nach Anspruch 1, **dadurch gekennzeichnet, daß** das Verschlußelement (10) eine im wesentlichen zylindrische Gestalt aufweist, wobei die Befestigungsmittel durch die Zylinderfläche gebildet werden und der schlitzförmige Einschnitt (141) in einer der Stirnflächen (14) des Zylinders angeordnet ist.

3. Medizinischer Artikel nach Anspruch 1 oder 2, **dadurch gekennzeichnet, daß** das Verschlußelement (10) symmetrisch ausgeführt ist.

4. Medizinischer Artikel nach einem oder mehreren der Ansprüche 1-3, **dadurch gekennzeichnet, daß** der schlitzförmige Einschnitt (141) kreuz- oder sternförmig ausgeführt ist.

5. Medizinischer Artikel nach einem oder mehreren der Ansprüche 1-4, **dadurch gekennzeichnet, daß** eine der den Einschnitt (141) aufweisenden Wandung (14) gegenüberliegende weitere Wandung (12) vorgesehen ist, die eine Öffnung (121) zum Durchtritt eines Fluids aufweist.

6. Medizinischer Artikel nach Anspruch 5, **dadurch gekennzeichnet, daß** der mittlere Bereich des Einschnittes (141) und der Öffnung (121) miteinander fluchten.

7. Medizinischer Artikel nach Anspruch 5 oder 6, **dadurch gekennzeichnet, daß** der sich um die Öffnung (121) der weiteren Wandung (12) erstreckende Bereich in einer im wesentlichen senkrecht zur Fügerichtung eines mit dem Anschluß (42) verbindbaren Konnektionselementes (50) verlaufenden Ebene angeordnet ist.

8. Medizinischer Artikel nach einem oder mehreren der Ansprüche 1-7, **dadurch gekennzeichnet, daß** das Verschlußelement (10) einteilig ausgeführt ist.

9. Medizinischer Artikel nach einem oder mehreren der Ansprüche 1-8, **dadurch gekennzeichnet, daß** die den Einschnitt (141) aufweisende Wandung (14) oder das gesamte Verschlußelement (10) aus Kunststoff bestehen.

10. Medizinischer Artikel nach Anspruch 9, **dadurch gekennzeichnet, daß** der Kunststoff Silikon ist.

11. Medizinischer Artikel nach einem oder mehreren der Ansprüche 1-10, **dadurch gekennzeichnet, daß** die den schlitzförmigen Einschnitt (141) aufweisende Wandung (14) des Verschlußelementes (10) ein in radialer Richtung wirkendes Federelement (145) aufweist.

12. Medizinischer Artikel nach Anspruch 11, **dadurch gekennzeichnet, daß** das Federelement (145) aus abwechselnd oder einzelnen auf der Ober- und/oder Unterseite der Wandung (14) angeordneten Vertiefungen (147) gebildet wird.

13. Medizinischer Artikel nach Anspruch 12, **dadurch gekennzeichnet, daß** die Vertiefungen (147) teilkreisförmig ausgeführt sind.

14. Medizinischer Artikel nach einem oder mehreren der Ansprüche 11-13, **dadurch gekennzeichnet, daß** in den sich in Längsrichtung des Einschnittes (141) erstreckenden Bereichen (149) der Wandung (14) kein Federelement vorgesehen ist.

15. Medizinischer Artikel nach einem oder mehreren der Ansprüche 1-14, **dadurch gekennzeichnet, daß** der schlitzförmige Einschnitt (141) des Verschlußelementes (10) bis zu einer Druckdifferenz von +/- 0,25 bar keimdicht schließt.

16. Verwendung eines Verschlußelementes (10) eines medizinischen Artikels in Form eines Filtermoduls für die Dialyse, Hämo- oder Ultrafiltration nach einem oder mehreren der Ansprüche 1-15, **dadurch gekennzeichnet, daß** das Verschlußelement (10) für die Inline-Sterilisation des Filtermoduls verwendet wird.

17. Verwendung nach Anspruch 16 , **dadurch gekennzeichnet, daß** das Befestigungsmittel des Verschlußelementes (10) derart ausgeführt ist, daß das Verschlußelement (10) auf vorstehende oder in buchsenförmige Anschlüsse auf- bzw. einsetzbar ist.

18. Medizinischer Artikel nach einem der vorangehenden Ansprüche, **dadurch gekennzeichnet, daß** das Verschlußelement (10) derart ausgeführt ist, daß die Innenfläche des Verschlußelementes (10) mit der Außenfläche eines vorstehenden Anschlusses (42) oder die Außenfläche des Verschlußelementes (10) mit der Innenfläche eines buchsenförmigen Anschlusses keimdicht verbunden sind.

19. Medizinischer Artikel nach einem der vorangehenden Ansprüche, **dadurch gekennzeichnet, daß** mindestens zwei Anschlüsse des medizinischen Artikels mit einem Verschlußelement nach einem oder mehreren der Ansprüche 1-15 versehen sind.

## Claims

1. A medical article comprising a filter module for dialysis, haemofiltration or ultrafiltration, comprising one or more connections (42) for the feed and/or discharge of a fluid, wherein at least one connection (42) is provided with a closure element (10), **characterised in that** the closure element (10) includes a wall (14) having an independently closing slot-shaped incision (141) which in the closed condition closes in germ-tight relationship, and that there are provided fixing means which adjoin the wall (14) and by means of which the closure element (10) can be sealingly connected to the connection (42).

2. A medical article according to claim 1 **characterised in that** the closure element (10) is of a substantially cylindrical configuration, wherein the fixing means are formed by the cylindrical surface and the slot-shaped incision (141) is arranged in one of the end faces (14) of the cylinder.

3. A medical article according to claim 1 or claim 2 **characterised in that** the closure element (10) is of a symmetrical configuration.

4. A medical article according to one or more of claims 1 to 3 **characterised in that** the slot-shaped incision (141) is of a cross-shaped or star-shaped configuration.

5. A medical article according to one or more of claims 1 to 4 **characterised in that** there is provided a further wall (12) which is in opposite relationship to the wall (14) having the incision (141) and which has an opening (121) for a fluid to pass through.

6. A medical article according to claim 5 **characterised in that** the central region of the incision (141) and the opening (121) are aligned with each other.

7. A medical article according to claim 5 or claim 6 **characterised in that** the region extending around the opening (121) of the further wall (12) is arranged in a plane extending substantially perpendicularly to the joining direction of a connection element (50) which can be connected to the connection (42).

8. A medical article according to one or more of claims 1 to 7 **characterised in that** the closure element (10) is of a one-piece nature.

9. A medical article according to one or more of claims 1 to 8 **characterised in that** the wall (14) having the incision (141) or the entire closure element (10) comprise plastic material.

10. A medical article according to claim 9 **characterised in that** the plastic material is silicone.

11. A medical article according to one or more of claims 1 to 10 **characterised in that** the wall (14) of the closure element (10), which has the slot-shaped incision (141), has a spring element (145) which acts in a radial direction.

12. A medical article according to claim 11 **characterised in that** the spring element (145) is formed from recesses (147) arranged alternately or individually on the top side and/or underside of the wall (14).

13. A medical article according to claim 12 **characterised in that** the recesses (147) are of a part-circular configuration.

14. A medical article according to one or more of claims 11 to 13 **characterised in that** no spring element is provided in the regions (149) of the wall (14), which extend in the longitudinal direction of the incision (141).

15. A medical article according to one or more of claims 1 to 14 **characterised in that** the slot-shaped incision (141) of the closure element (10) closes in germ-tight relationship up to a pressure difference of +/-0.25 bar.

16. Use of a closure element (10) of a medical article in the form of a filter module for dialysis, haemofiltration or ultrafiltration, according to one or more of claims 1 to 15, **characterised in that** the closure element (10) is used for inline sterilisation of the filter module.

17. Use according to claim 16 **characterised in that** the fixing means of the closure element (10) are such that the closure element (10) can be fitted on to or into projecting or socket-shaped connections.

18. A medical article according to one of the preceding claims **characterised in that** the closure element (10) is such that the inside surface of the closure element (10) is connected in germ-tight relationship to the outside surface of a projecting connection (42) or the outside surface of the closure element (10) is connected in germ-tight relationship to the inside surface of a socket-shaped connection.

19. A medical article according to one of the preceding claims **characterised in that** at least two connections of the medical article are provided with a closure element according to one or more of claims 1 to 15.

## Revendications

1. Article médical constitué d'un module filtrant pour la dialyse, l'hémo- ou l'ultra-filtration avec un ou plusieurs raccords (42) pour l'amenée et/ou l'évacuation d'un fluide, où au moins un raccord (42) est muni d'un élément de fermeture (10), **caractérisé en ce que** l'élément de fermeture (10) comprend une paroi (14) qui présente une entaille en forme de fente (141) se fermant automatiquement qui, à l'état fermé, ferme d'une manière étanche aux germes, et **en ce que** des moyens de fixation sont prévus qui avoisinent la paroi (14) et au moyen desquels l'élément de fermeture (10) peut être relié d'une manière étanche au raccord (42).

2. Article médical selon la revendication 1, **caractérisé en ce que** l'élément de fermeture (10) présente une forme sensiblement cylindrique, où les moyens de fixation sont formés par la face cylindrique, et l'entaille en forme de fente (141) est disposée dans l'une des faces frontales (14) du cylindre.

3. Article médical selon la revendication 1 ou 2, **caractérisé en ce que** l'élément de fermeture (10) est réalisé d'une manière symétrique.

4. Article médical selon l'une ou plusieurs des revendications 1 à 3, **caractérisé en ce que** l'entaille en forme de fente (141) est réalisée en forme de croix ou d'étoile.

5. Article médical selon l'une ou plusieurs des revendications 1 à 4, **caractérisé en ce qu'**une paroi supplémentaire (12) opposée à la paroi (14) présentant l'entaille (141) est prévue, qui présente une ouverture (121) pour le passage d'un fluide.

6. Article médical selon la revendication 5, **caractérisé en ce que** la zone médiane de l'entaille (141) et de l'ouverture (121) sont alignées.

7. Article médical selon la revendication 5 ou 6, **caractérisé en ce que** la zone s'étendant autour de l'ouverture (121) de la paroi supplémentaire (12) est disposée dans un plan s'étendant sensiblement perpendiculairement à la direction d'assemblage d'un élément de connexion (50) pouvant être relié au raccord (42).

8. Article médical selon l'une ou plusieurs des revendications 1 à 7, **caractérisé en ce que** l'élément de fermeture (10) est réalisé en une pièce.

9. Article médical selon l'une ou plusieurs des revendications 1 à 8, **caractérisé en ce que** la paroi (14) présentant l'entaille (141) ou bien l'ensemble de l'élément de fermeture (10) sont réalisés en matériau synthétique.

10. Article médical selon la revendication 9, **caractérisé en ce que** le matériau synthétique est du silicone.

11. Article médical selon l'une ou plusieurs des revendications 1 à 10, **caractérisé en ce que** la paroi (14) de l'élément de fermeture (10) présentant l'entaille en forme de fente (141) comporte un élément à ressort (145) agissant dans la direction radiale.

12. Article médical selon la revendication 11, **caractérisé en ce que** l'élément à ressort (145) est formé par des creux (147) disposés alternativement ou individuellement sur le côté supérieur et/ou inférieur de la paroi (14).

13. Article médical selon la revendication 12, **caractérisé en ce que** les creux (147) sont réalisés en forme de cercle partiel.

14. Article médical selon l'une ou plusieurs des revendications 11 à 13, **caractérisé en ce qu'**un élément à ressort n'est pas prévu dans les zones (149) de la paroi (14) s'étendant dans la direction longitudinale de l'entaille (141).

15. Article médical selon l'une ou plusieurs des revendications 1 à 14, **caractérisé en ce que** l'entaille en forme de fente (141) de l'élément de fermeture (10) ferme d'une manière étanche aux germes jusqu'à une différence de pression de +/-0,25 bar.

16. Utilisation d'un élément de fermeture (10) d'un article médical sous la forme d'un module filtrant pour la dialyse, l'hémo- ou l'ultra-filtration selon l'une ou plusieurs des revendications 1 à 15, **caractérisée en ce que** l'élément de fermeture (10) est utilisé pour la stérilisation en ligne du module filtrant.

17. Utilisation selon la revendication 16, **caractérisée en ce que** le moyen de fixation de l'élément de fermeture (10) est réalisé de façon que l'élément de fermeture (10) puisse être placé sur ou dans des raccords saillants ou en forme de douille.

18. Article médical selon l'une des revendications précédentes, **caractérisé en ce que** l'élément de fermeture (10) est réalisé de façon que la face intérieure de l'élément de fermeture (10) soit reliée d'une manière étanche aux germes à la face extérieure d'un raccord saillant (42), ou bien la face extérieure de l'élément de fermeture (10) à la face intérieure d'un raccord en forme de douille.

19. Article médical selon l'une des revendications précédentes, **caractérisé en ce qu'**au moins deux raccords de l'article médical sont pourvus d'un élément de fermeture selon l'une ou plusieurs des revendications 1 à 15.
